(19) Europäisches Patentamt
European Patent Office
Office européen des brevets

(11) Veröffentlichungsnummer: **0 186 049**
**B1**

(12) **EUROPÄISCHE PATENTSCHRIFT**

(45) Veröffentlichungstag der Patentschrift: **04.07.90**

(21) Anmeldenummer: **85115785.9**

(22) Anmeldetag: **11.12.85**

(51) Int. Cl.$^5$: **C 07 D 401/06,** A 61 K 31/44
// C07D213/79, C07D213/85

(54) **N-substituierte Aziridin-2-Carbonsäurederivate, Verfahren zu deren Herstellung sowie diese Substanzen enthaltende Arzneimittel.**

(30) Priorität: **21.12.84 DE 3446713**

(43) Veröffentlichungstag der Anmeldung:
**02.07.86 Patentblatt 86/27**

(45) Bekanntmachung des Hinweises auf die Patenterteilung:
**04.07.90 Patentblatt 90/27**

(84) Benannte Vertragsstaaten:
**AT BE CH DE FR GB IT LI LU NL SE**

(56) Entgegenhaltungen:
**GB-A-2 026 863**

**CHEMISCHE BERICHTE, Band 105, Nr. 1, 1972, Seiten 312-324; K.-D. GUNDERMANN et al.: "Verbesserte Synthese von 2-Cyan-aziridinen und Untersuchungen zu ihrer Isomerisierung"**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH**
**Sandhofer Strasse 116**
**D-6800 Mannheim 31 (DE)**

(72) Erfinder: **Bosies, Elmar, Dr.rer.nat.**
**Delpstrasse 11**
**D-6940 Weinheim (DE)**
Erfinder: **Endele, Richard, Dr.rer.nat.**
**Richard-Wagner-Strasse 39b**
**D-6901 Wilhelmsfeld (DE)**
Erfinder: **Pahlke, Wulf, Dr.**
**Arminstrasse 37**
**D-6140 Bensheim (DE)**

**EP 0 186 049 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

In der DE—OS 28 33 986 sind N-alkylierte Aziridin-2-carbon-säurederivate mit immunstimulierender Wirkung beschrieben, u.a. auch das 2-Cyan-1-[(2-methoxy-6-methyl-3-pyridinyl)methyl]aziridin. Es wurde nun gefunden, daß auch andere N-pyridinyl-methyl-aziridincarbonsäurederivate, die teilweise Metabolite der genannten Verbindung sind, Verbindungen mit guter immunstimulierender Wirkung darstellen.

Zusätzlich zeigen die neuen Verbindungen eine hervorragende immunsuppressive Wirkung, so daß sie als Immunmodulatoren Verwendung finden können.

Diese Verbindungen sind wirkungsvoll für die Behandlung von Krankheiten, bei denen der erhöhte Grad der Antikörperproduktion oder der Monocyten/Lymphocyten-Aktivität als Ergebnis der Hyperreaktivität des immunregulatorischen Netzwerkes eng mit der Entwicklung von Autoimmum-Krankheiten verbunden ist, einschließlich rheumatischer Arthritis [Mellbye, O J. and Natvig, J. B. Clin, Exp. Immunol., *8*, 889 (1971)], multiple Sklerose [Tourtellotte, W. W. and Parker, J. A. Science, 154, 1044 (1966)], Lupus erythematodes [Abdou, N. I., *et al.*, Clin. Immunol, Immunopath., *6*, 192 (1976)], Thyroiditis [Witebsky, E., *et al.*, J. Immunol., *103*, 708 (1969)], Gewebekrankheiten [Sharp, G. C., *et al.*, Am. J. Med., *52*, 148 (1972)], Haut-Polymigositis [Venables, P. J. W., *et al.*, Ann. Rheum. Dis., *40*, 217 (1981)], insulinabhängiger Diabetes [Charles, M. A., *et al.*, J. Immunol., 130, 1189, (1983)] und bei Patienten mit Organtransplantationen.

Gegenstand der Erfindung sind Aziridin-2-carbonsäurederivate der allgemeinen Formel (I)

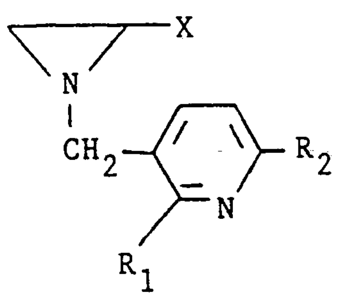

(I)

in der

X eine Nitrilgruppe

$R_1$ eine $C_1$—$C_4$-Alkoxygruppe,

$R_2$ der Hydroxymethyl-, der Carboxyl-, der $C_2$—$C_5$-Alkoxycarbonyl-, oder Formyl- oder der Carbamoylrest ist,

deren Salze und N-Oxide, sowie die Herstellung und Verwendung derselben zur Herstellung von Arzneimitteln mit immunmodulierender Wirkung.

Gegenstand der Erfindung sind ferner sämtliche stereoisomeren Verbindungen, die z.B. auf Grund asymmetrischer Kohlenstoffatome anfallen, wobei die Trennung der stereoisomeren Formen nach an sich bekannten Verfahren vorgenommen wird, sowie ferner deren Verwendung zur Herstellung von Immunmodulatoren.

Unter $C_1$—$C_4$-Alkoxy versteht man Reste mit 1—4 Kohlenstoffatomen, vorzugsweise Methoxy und Ethoxy. Als $C_2$—$C_5$-Alkoxycarbonylreste $R_2$ sind Reste mit 2—5 Kohlenstoffatomen, insbesondere der Methoxycarbonyl- und Ethoxycarbonylrest, anzusehen.

Verbindungen der allgemeinen Formel I lassen sich nach an sich bekannten Verfahren darstellen, vorzugsweise, indem man

a) eine Verbindung der allgemeinen Formel II

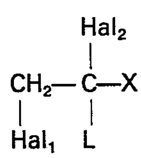

(II),

in der X die oben angegebene Bedeutung hat, $Hal_1$ und $Hal_2$ Chlor oder Brom sind, L Wasserstoff bedeutet, woebei $Hal_1$ und L zusammen auch ein Valenzstrich sein können, mit einem Amin der allgemeinen Formel III oder dessen Pyridin-N-oxid

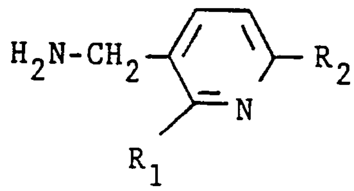

(III)

in der $R_1$ und $R_2$ die oben angegebenen Bedeutungen haben, umsetzt oder

b) eine Verbindung der allgemeinen Formel IV, deren Salz oder deren Pyridin-N-oxid

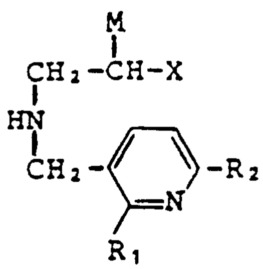

bzw.

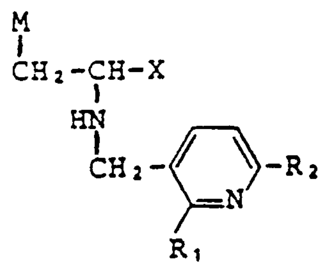

(IV),

in der X, $R_1$ und $R_2$ die oben angegebenen Bedeutungen haben und M Chlor, Brom oder die Gruppe A—Z bedeuten soll, wobei A Sauerstoff oder Schwefel und Z Wasserstoff oder eine zusammen mit Sauerstoff bzw. Schwefel leicht eliminierbare Gruppierung darstellt, mit einem M-H-abspaltenden Reagenz behandelt oder

c) eine Verbindung der allgemeinen Formel V

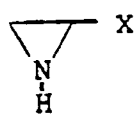

(V),

in der X die oben angegebene Bedeutung hat, mit einer Verbindung der allgemeinen Formel VI oder deren N-Oxid

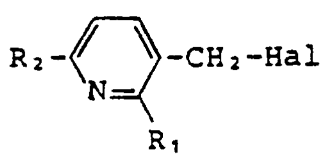

in der $R_1$ und $R_2$ die oben angegebenen Bedeutungen haben und Hal, Chlor, Brom oder Iod darstellt, umsetzt oder

d) ein Azid der allgemeinen Formel VII oder dessen N-Oxid

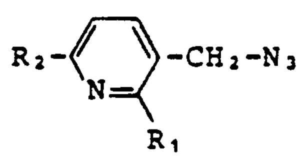

(VII),

in der $R_1$ und $R_2$ die oben angegebenen Bedeutungen haben, mit einer Verbindung der allgemeinen Formel VIII

$$CH_2{=}CH{-}X \qquad (VIII),$$

in der X die oben angegebene Bedeutung hat, zu einer Verbindung der allgemeinen Formel I umsetzt, wobei als Zwischenstufe ein Triazolin der allgemeinen Formel IX oder dessen Pyridin-N-oxid

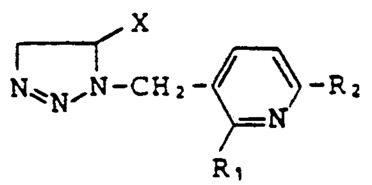 bzw. 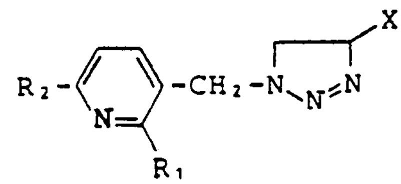 (IX),

in der $R_1$, $R_2$ und X die oben angegebenen Bedeutungen haben, entsteht, das durch Thermolyse oder Photolyse unter Stickstoffabspaltung in eine Verbindung der allgemeinen Formel I umgewandelt wird oder

e) ein Epoxid der allgemeinen Formel X

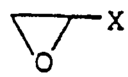 (X),

in der X die oben angegebene Bedeutung hat, mit einem Amin der allgemeinen Formel III oder dessen Pyridin-N-oxid umsetzt oder

f) ein Oxazolidinon der allgemeinen Formel XI oder dessen Pyridin-N-oxid

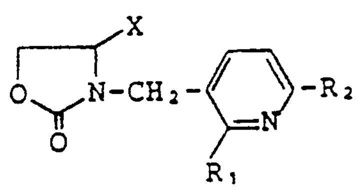 bzw. 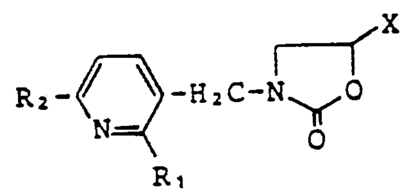 (XI),

in der $R_1$, $R_2$ und X die oben angegebenen Bedeutungen haben, einer Thermolyse unterwirft oder

g) eine Verbindung der allgemeinen Formel XII oder deren Pyridin-N-oxid

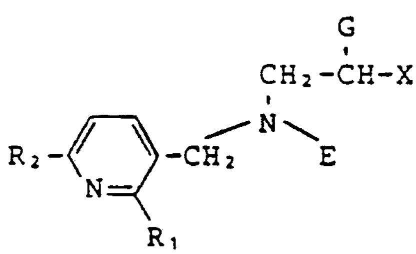 bzw. 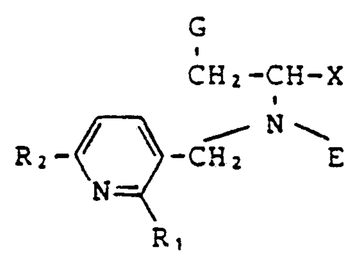 (XII),

in der $R_1$, $R_2$ und X die oben angegebenen Bedeutungen haben und G Wasserstoff oder Hal und E Hal, eine Trialkylaminogruppe oder einen Arylsulfonsäureesterrest bedeuten, wobei Hal Chlor oder Brom ist, mit einem E-G-abspaltenden Reagenz behandelt, wobei man die so erhaltenen Verbindungen der allgemeinen Formel I nachträglich in andere Verbindungen der allgemeinen Formel I umwandelt, sowie gewünschtenfalls die Verbindungen der allgemeinen Formel I in ihre pharmakologisch unbedenklichen Salz überführt.

Das Verfahren a) zur Herstellung der Aziridinderivate der allgemeinen Formel I ist literaturbekannt [s. z.B. GUNDERMANN et al., Chem. Ber. *105*, 312 (1972) und WAGNER-JAUREGG, Helv. Chim. Acta *44*, 1237 (1961)]. Bevorzugt werden hierbei inerte Lösungsmittel eingesetzt, wie z.B. Ether, Dioxan, Benzol oder Toluol, man kann jedoch auch niedere Alkohole wie Methanol oder Ethanol verwenden. Die Reaktionstemperaturen liegen zwischen 0 und 80°C, vorzugsweise wird bei Zimmertemperatur gearbeitet. Die Reaktionszeit schwankt zwischen 3 h und 10 Tagen.

Beim Verfahren b) setzt man als M-H-abspaltendes Reagenz Basen ein, vor allem tert. Amine wie Triethylamin, Triethanolamin oder Dicyclohexylethylamin Hierbei kann man inerte Lösungsmittel wie Ether, Dioxan, Benzol oder Toluol aber auch sehr gut Alkohole wie Methanol oder Ethanol verwenden. Darüberhinaus finden in einigen Fällen vor allem Alkoholate wie Natriummethylat oder Natriumethylat in dem entsprechenden Alkohol Anwendung. Bewährt hat sich, vor allem, wenn die Gruppe A—Z die OH-Gruppe bedeutet, als wasserabspaltendes Mittel Triphenylphosphin in Gegenwart von Tetrachlorkohlenstoff und Triethylamin, wobei dann in der Regel Methylenchlorid oder Chloroform als Lösungsmittel eingesetzt wird. Diese Wasserabspaltung gelingt jedoch auch mit Schwefelsäure. Die Reaktionszeiten liegen zwischen 3 und 24 h.

Die Alkylierungsreaktion bei Verfahren c) wird bevorzugt in Wasser, Alkoholen wie Methanol und Ethanol oder in Alkohol/Wasser-Gemischen in Gegenwart einer Base durchgeführt. Neben organischen Basen kann man sehr gut auch anorganische Basen, wie z.B Alkalicarbonate oder Alkalibicarbonate als Säureacceptoren einsetzen. Die Umsetzungen werden in der Regel bei Temperaturen von 20—60°C

durchgeführt. Zur Beschleunigung der Reaktion kann man gegebenenfalls Phasentransferkatalysatoren, wie. z.B. Triethylbenzylammoniumchlorid, zusetzen.

Die Thermolyse der Triazoline beim Verfahren d) wird bei 80—150°C, vorzugsweise bei 100—120°C durchgeführt. Man kann ohne Lösungsmittel arbeiten und die entstandenen Aziridinderivate durch Destillation oder Umkristallisation reinigen. Ebensogut kann man jedoch auch ein Lösungsmittel verwenden, wobei sich inerte Lösungsmittel, wie z.B. Benzol, Toluol und Xylol besonders bewährt haben. Photolysen werden in der Regel bei Zimmertemperatur in Lösung vorgenommen, wobei hier vor allem Benzol, Toluol oder auch Acetonitril verwendet werden. Die Photolysen können mit oder ohne Sensibilisatoren, wie z.B. Benzochinon oder Acetophenon durchgeführt werden [s. z.B. Am. Chem. Soc. *90*, 988 (1968)].

Beim Verfahren e) kann man ein Epoxid der allgemeinen Formel X mit einem Amin der allgemeinen Formel III oder dessen Pyridin-N-oxid reagieren lassen und den dabei entstehenden Aminoalkohol wie beim Verfahren b) beschrieben zu einem Aziridinderivat der allgemeinen Formel I dehydratisieren.

Oxazolidinone der allgemeinen Formel XI werden in der Regel ohne Lösungsmittel in Gegenwart von Basen, wie z.B. Triethanolamin oder Dicylohexylethylamin thermolysiert. Die Thermolysetemperaturen liegen zwischen 170 und 250°C.

Beim Verfahren g) werden als E-G-abspaltende Reagenzien im Falle, daß G gleich Wasserstoff ist, bevorzugt Alkoholate wie Alkalimethylat oder Alkaliethylat in den entsprechenden Alkoholen eingestezt. Mann kann jedoch auch tert. Amine wie Triethylamin, Triethanolamin oder Dicylohexylethylamin in Lösungsmitteln wie Methanol, Ethanol, Benzol, Toluol, Ether oder Dioxan verwenden. Für den Fall, daß G und E gleich Hal sind, kann man zur Abspaltung gängige Enthalogenierungsmittel, vorzugsweise Zink oder Natrium, verwenden.

Die nachträgliche Umwandlung von Verbindungen der allgemeinen Formel I in andere Verbindungen der allgemeinen Formel I kann einerseits durch Umwandlung der Substituenten X und $R_2$ geschehen. Hierbei können z.B. Verbindungen mit X bzw. $R_2$ = $C_2$—$C_5$-Alkoxycarbonyl durch Umsetzung mit Ammoniak in Verbindungen mit X bzw. $R_2$ = Carbamoyl überführt werden, wobei diese im Falle mit X = Carbamoyl wiederum mit Dehydratisierungsmitteln in Verbindungen mit X = Nitril überführt werden können.

Verbindungen der allgemeinen Formel I, in der X = $C_2$—$C_5$-Alkoxycarbonyl oder Carbamoyl bedeuten, können daher auch als Zwischenprodukte zur Herstellung von Verbindungen der allgemeinen Formel I, in der X = Nitril bedeutet, verwendet werden.

Verbindungen der allgemeinen Formel I, in der $R_2$ die Hydroxymethylgruppe bedeutet, lassen sich durch Reduktion der entsprechenden Verbindung mit $R_2$ = Carboxyl, Formyl bzw. $C_2$—$C_5$-Alkoxycarbonyl darstellen.

Verbindungen der allgemeinen Formel I mit $R_2$ = Carboxyl lassen sich durch Oxidation von Verbindungen der allgemeinen Formel I mit $R_2$ = Hydroxymethyl oder Formyl bzw. durch Verseifung von Verbindungen der allgemeinen Formel I mit $R_2$ = $C_2$—$C_5$-Alkoxycarbonyl bzw. Carbamoyl herstellen.

Verbindungen der allgemeinen Formel I, in der $R_2$ = $C_2$—$C_5$-Alkoxycarbonyl, Formyl oder Carbamoyl bedeuten, können daher auch als Zwischenprodukte zur Herstellung von Verbindungen der allgemeinen Formel I, in der $R_2$ die Hydroxymethyl- bzw. die Carboxylgruppe bedeutet, verwendet werden.

Die nachträgliche Umwandlung von Verbindungen der allgemeinen Formel I in andere Verbindungen der allgemeinen Formel I kann andererseits auch durch die Überführung in die entsprechenden N-Oxide bzw. in der Reduktion der N-Oxide zu den entsprechenden Stickstoffbasen geschehen. Die Oxidation zu den N-Oxiden geschieht nach literaturbekannten Methoden, vorzugsweise durch Umsetzung mit einem Peroxid, wie z.B. Wasserstoffperoxid, wobei in der Regel Essigsäure als Lösungsmittel verwendet wird. Die Reduktion des N-Oxids führt man üblicherweise durch Hydrierung mit Raney-Nickel als Katalysator durch. Als Lösungsmittel setzt man hierbei bevorzugt niedere Alkohole wie Methanol oder Ethanol ein.

Die Umwandlung der Ester- in die Amid-Gruppierung läßt sich mit gasförmigem Ammoniak in einem organischen Lösungsmittel, vorzugsweise in Methanol oder Ethanol, oder wässerigem Ammoniak bei 0 bis +25°C durchführen. Das gewünschte Amid fällt aus oder wird aus dem Reaktionsgemisch z.B. durch Säulenchromatographie isoliert.

Zur Umwandlung der Carbamoyl- in die Nitrilgruppe werden literaturbekannte Dehydratisierungsmittel eingesetzt, wobei vor allem das Gemisch aus Triphenylphosphin, Tetrachlorkohlenstoff und Triethylamin angewendet wird. Als Lösungsmittel nimmt man üblicherweise halogenierte Kohlenwasserstoffe wie z.B. Methylenchlorid bzw. Chloroform oder aber auch Acetonitril.

Die Umwandlung der Verbindungen der allgemeinen Formel I mit einer $C_2$—$C_5$-Alkoxycarbonyl-, Carbamoyl- oder Cyangruppe in eine Carboxylverbindung erfolgt in der Regel durch Verseifung nach in der Literatur beschriebenen Verfahren.

Die Umwandlung einer $C_2$—$C_5$-Alkoxycarbonyl-, Formyl bzw. Carboxyl- in die Hydroxymethylgruppe geschieht vorzugsweise mit Borhydriden wobei die Carboxylgruppe gegebenenfalls vorher z.B. durch Umsetzung zu einem gemischten Anhydrid aktiviert werden kann. Man kann z.B. Lithiumborhydrid, Natriumborhydrid/Lithiumchlorid, Natriumborhydrid in Polyethylenglykol, Natriumborhydrid/Aluminiumchlorid oder Boran/Tetrahydrofuran einsetzen. Als Lösungsmittel finden vor allem niedere Alkohole wie Methanol oder Ethanol, Tetrahydrofuran, Diethylenglykoldimethylether oder

Polyethylenglykol Verwendung. Die Reduktionen werden in einem Temperaturbereich von 0—60°C, vorzugsweise bei 20—30°C durchgeführt.

Die Oxidation der Hydroxymethyl- oder Formyl gruppe in die Carboxylgruppe lässt sich mit gängigen Oxidationsmitteln wie z.B. Kaliumpermanganat, Tetrabutylammoniumpermanganat, Bis(tetrabutylammonium)dichromat, Nickelperoxid oder Pyridiniumdichromat durchführen. Als Lösungsmittel verwendet man hierbei je nach Oxidationsmittel Wasser, halogenierte Kohlenwasserstoffe, Ether oder Dimethylformamid, wobei die Reaktionen bei Temperaturen von 20—100°C durchgeführt werden.

Zur Herstellung pharmazeutischer Mittel mit immunmodulierender Wirkung werden die Verbindungen der allgemeinen Formel I in an sich bekannter Weise mit geeigneten pharmazeutischen Trägersubstanzen gemischt und beispielsweise als Tabletten oder Dragees ausgeformt oder unter Zugabe entsprechender Hilfsstoffe in Wasser oder Öl, wie z.B. Olivenöl, suspendiert oder gelöst und in Steckkapseln abgefüllt.

Da der Wirkstoff säurelabil ist, wird die Zubereitung mit einem erste im alkalischen Dünndarmmilieu löslichen Überzug versehen oder ein entsprechender Trägerstoff, wie beispielsweise eine höhere Fettsäure oder Carboxymethylcellulose, zugemischt. Feste Trägerstoffe sind z.B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole), für orale Applikation geeignete Zubereitungen können gewünschtenfalls Geschmacks- und Süßstoffe enthalten.

Als Injektionsmedium kommt vorzugsweise Wasser zur Anwendung, welches die bei Injektionslösungen üblichen Zusätzte wie Stabilisierungsmittel, Lösungsvermittler oder schwach alkalische Puffer enthält. Derartige Zusätze sind z.B. Dimethylsulfoxyd, Dimethylformamid, N-Methylpyrrolidon, Phosphat- oder Carbonatpuffer, Ethanol, Komplexbildner (wie Ethylendiamintetraessigsäure und deren nicht-toxische Salze), hochmolekulare Polymere (wie flüssiges Polyethylenoxyd) zur Viskositätsregulierung.

Zur Bekämpfung von Krankheiten, die mit einer Schwächung des Immunsystems verbunden sind, werden die pharmakologisch aktiven Verbindungen der allgemeinen Formel I in Einzeldosen von 1 bis 600, vorzugsweise von 50 bis 500 mg angewandt, wobei diese Einzeldosen je nach Bedarf ein- oder mehrmals pro Tag verabreicht werden können.

Bevorzugt im Sinne der Erfindung können außer den in den Beispielen genannten Verbindungen noch die folgenden Verbindungen zur Herstellung von Arzneimitteln mit immunmodulierender Wirkung verwendet werden:

Die nachfolgenden Beispiele zeigen einige der zahlreichen Verfahrensvarianten, die zur Synthese der erfindungsgemaeßen Verbindungen verwendet werden können.

Die Struktur aller in den nachfolgenden Beispielen beschriebenen Substanzen sind durch Mikroverbrennungsanalyse, NMR- und Massenspektren gesichert.

## Beispiel 1

5-[(2-Cyan-1-aziridinyl)methyl]-6-methoxy-pyridin-2-carbonsäure (Natriumsalz)

2.33 g (44 mmol) Acrylnitril verdünnt man mit 1 ml Diethylether und läßt unter Rühren und Belichten 7.05 g (44 mmol) Brom zutropfen. Durch gelegentliches Kühlen erreicht man, daß die Innentemperatur 40°C nicht übersteigt. Man läßt 5 min weiterrühren, verdünnt mit 60 ml Methanol und tropft eine Lösung von 6.55 g (44 mmol) Triethanolamin in 60 ml Methanol zu. Nach einer weiteren Stunde tropft man gleichzeitig eine Lösung von 6.55 g (44 mmol) Triethanolamin in 60 ml Methanol und eine Lösung von 8 g (44 mol) 5-Aminomethyl-6-methoxy-pyridin-2-carbonsäure (Fp: 269—270°C Z.) In 1 Liter Methanol/200 ml Wasser zu. Man läßt die Lösung 24 Stunden stehen, engt weitgehend am Rotationsverdampfer ein, bringt die wässerige Lösung mit 2 N Salzsäure auf einen pH-Wert von 4.5 und extrahiert kontinuierlich 2 Tage mit Methylenchlorid. Die Methylenchloridphase wird getrocknet und eingeengt. Mann erhält so 4.8 g eines Öls, das in 50 ml Ethanol gelöst wird. Unter Kühlung versetzt man die Lösung mit einer Lösung von 440 mg Natrium in 15 ml Ethanol.

Das Natriumsalz der 5-[(2-Cyan-1-aziridinyl)methyl]-6-methoxy-pyridin-2-carbonsäure fällt aus, wird nach Zugabe von 100 ml Isopropanol abgesaugt, getrocknet und mit Diethylether gewaschen. Man erhählt 3.7 g = 33% der Theorie der gewünschten Verbindung mit einem Fp: 230—232°C.

Aus den Methylenchloridextrakten kann man nach Abziehen des Methylenchlorids und Aufnehmen des öligen Rückstandes in wenig Isopropanol mit Diethylether die 5-[(2-Cyan-1-aziridinyl)methyl]-6-methoxy-pyridin-2-carbonsäure mit einem Fp: 96—97°C isolieren.

Die als Ausgangsmaterial eingesetzte 5-Aminomethyl-6-methoxy-pyridin-2-carbonsäure erhält man auf folgende Weise:

3-Cyan-2-methoxy-6-methylpyridin wird mit Kaliumpermanganat in wässriger Lösung zur 5-Cyan-6-methoxy-pyridin-2-carbonsäure (Fp: 235—237°C) oxidiert. Anschließende Hydrierung an Palladium/Kohle in Isopropanol/Wasser/Salzsäure führt zu der gewünschten Verbindung.

In analoger Weise erhält man durch Umsetzung des durch Bromierung von Acrylnitril und Bromwasserstoffabspaltung entstandenen 2-Bromacrylnitrils mit.

a) 5-Aminomethyl-6-methoxy-pyridin-2-carbonsäuremethylester (Fp des HCl-Salzes: 224—226°C) den 5-[(2-Cyan-1-aziridinyl)methyl]-6-methoxy-pyridin-2-carbonsäuremethylester; Fp (aus Isopropanol): 156—158°C) Ausbeute: 57%

Den als Ausgangsmaterial eingesetzten 5-Aminomethyl-6-methoxy-pyridin-2-carbonsäuremethylester stellt man auf folgende Weise her:

5-Cyan-6-methoxy-pyridin-2-carbonsäure (s.S. 15) wird in methanol mit Hilfe von Trimethylchlorsilan zum 5-Cyan-6-methoxy-pyridin-2-carbonsäuremethylester (Fp: 124—125°C) umgesetzt. Die anschließende Hydrierung in Methanol an Palladium/Kohle in Gegenwart von Salzsäure ergibt die gewünschte Aminomethylverbindung (Fp. des HCl-Salzes: 224—226°C). Die Hydrierlösung wird durch Zugabe von wenigen Tropfen Triethanolamin auf einen pH Wert von 7 gebracht und direkt für die folgende Reaktion verwendet.

Beispiel 2

1-[(6-Hydroxymethyl-2-methoxy-3-pyridinyl)methyl]-2-aziridin-carbonitril

247 mg (1 mmol) 5-[(2-Cyan-1-aziridinyl)methyl]-6-methoxy-pyridin-2-carbonsäuremethylester (s. Beispiel 1) löst man in 10 ml absolutem Tetrahydrofuran und versetzt die Lösung mit 74 mg (2 mmol) Natriumborhydrid und 84 mg (2 mmol) Lithiumchlorid. Nach zwölfstündigem Rühren bei Zimmertemperatur gibt man noch einmal dieselben Mengen Natriumborhydrid und Lithiumchlorid zu und versetzt nach weiteren 12 Stunden mit 5 ml Wasser. Man engt ein, nimmt den Rückstand in Ethanol auf, filtriert, engt das Filtrat ein, nimmt den Rückstand in Aceton auf, filtriert und engt ein. Den Rückstand reinigt man über eine Kieselgelsäule (Fließmittel: Aceton/Toluol i.V. 1/3; Rf-Wert ca. 0.3). Man erhält 120 mg = 55% der Theorie mit einem Fp: 68—70°C.

Beispiel 3

5-[(2-Cyan-1-aziridinyl)methyl]-6-ethoxy-pyridin-2-carbonsäure

In analoger Weise — wie in Beispiel 1 beschrieben — erhält man durch Umsetzung des durch Bromierung von Acrylnitril und Bromwasserstoffabspaltung entstandenen 2-Bromacrylnitrils mit 5-Aminomethyl-6-ethoxy-pyridin-2-carbonsäure (Fp: 226—228°C) die 5-[(2-Cyan-1-aziridinyl)methyl]-6-ethoxy-pyridin-2-carbonsäure (Fp: 108—111°C) in einer Ausbeute von 35%.

Die als Ausgangsmaterial verwendete 5-Aminomethyl-6-ethoxy-pyridin-2-carbonsäure stellt man auf folgende Weise her:

2-Chlor-6-methyl-pyridin-2-carbonitril wird mit Natriumethylat in Ethanol zum 2-Ethoxy-6-methyl-pyridin-3-carbonitril (Fp: 90—92°C) umgesetzt und anschließend mit Kaliumpermanganat zur 5-Cyan-6-ethoxy-pyridin-2-carbonsäure (Fp: 185—190°C) oxidiert. Durch katalytische Hydrierung an Palladium/Kohle erhält man dann die 5-Aminomethyl-6-ethoxy-pyridin-2-carbonsäure.

Beispiel 4

5-[(2-Cyan-1-aziridinyl)methyl]-6-methoxy-pyridin-2-carboxaldehyd

2.4 g des in Beispiel 2 beschriebenen 1-[(6-Hydroxymethyl-2-methoxy-3-pyridinyl)methyl]-2-aziridin-carbonitrils werden in 80 ml Methylenchlorid gelöst und mit 6 g aktiviertem Braunstein versetzt. Man rührt 24 h bei Zimmertemperatur, gibt noch einmal dieselbe Menge aktivierten Braunstein zu und läßt wieder 24 h bei Zimmertemperatur rühren. Anschließend wird abgesaugt, das Filtrat eingeengt und der Rückstand über eine Kieselgelsäule gereinigt (Elutionsmittel: Dioxan/Ligroin i.V 1/3). Die Fraktion mit der gewünschten Verbindung werden eingeengt und der Rückstand aus Toluol umkristallisiert.

Ausbeute: 1.4 g = 59% der Theorie, Fp. 104—105°C.

**Patentansprüche**

1. Verbindungen der allgemeinen Formel I

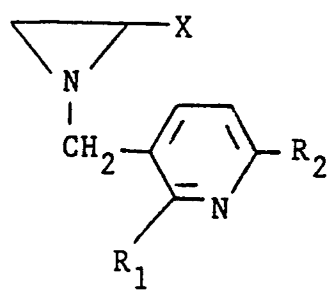

(I)

in der

X eine Nitrilgruppe

$R_1$ eine $C_1$—$C_4$-Alkoxygruppe,

$R_2$ der Hydroxymethyl-, der Carboxyl-, der $C_2$—$C_5$-Alkoxycarbonyl-, der Formyl- oder der Carbamoylrest ist, deren Salze und N-Oxide.

2. Verbindungen gemäß Anspruch 1 betreffend 5-[(2-Cyan-1-aziridinyl)methyl]-6-methoxy-pyridin-2-carbonsäure und 1-[(6-Hydroxymethyl-2-methoxy-3-pyridinyl)methyl]-2-aziridin-carbonitril.

3. Verfahren zur Herstellung von Verbindungen der allgemeinen Formel I

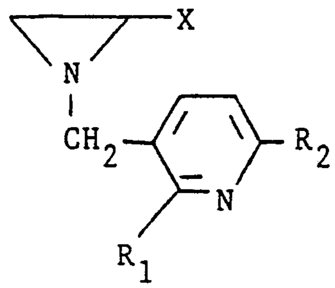

(I)

in der

X eine Nitrilgruppe

$R_1$ eine $C_1$—$C_4$-Alkoxygruppe,

$R_2$ der Hydroxymethyl-, der Carboxyl-, der $C_2$—$C_5$-Alkoxycarbonyl-, der Formyl- oder der Carbamoylrest ist, deren Salze und N-Oxide, dadurch gekennziechnet, daß man nach an sich bekannten Methoden eine Verbindung der allgemeinen Formel II

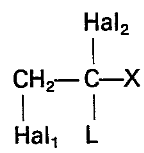

(II),

in der X die oben angegebene Bedeutung hat, $Hal_1$ und $Hal_2$ Chlor oder Brom sind, L Wasserstoff bedeutet, wobei $Hal_1$ und L zusammen auch ein Valenzstrich sein können, mit einem Amin der allgemeinen Formel III oder dessen Pyridin-N-oxid

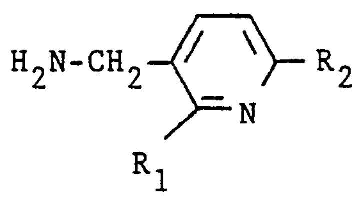

(III)

in der $R_1$ und $R_2$ die oben angegebenen Bedeutungen haben umsetzt und anschließend die so erhaltene Verbindung der allgemeinen Formel I nachträglich in andere Verbindungen der allgemeinen Formel I umwandelt, sowie gewünschtenfalls die Verbindung der allgemeinen Formel I in ihre pharmakologisch unbedenklichen Salze überfürt.

4. Arzneimittel, enthaltend eine der Verbindungen gemäß Anspruch 1 oder 2 neben üblichen Träger- und Hilfsstoffen.

5. Verwendung der Verbindungen gemäß Anspruch 1 oder 2 zur Herstellung von Arzneimitteln für die Bekämpfung von mit einer Schwächung des Immunsystems verbundenen Krankheiten.

**Revendications**

1. Composés répondant à la formule générale I

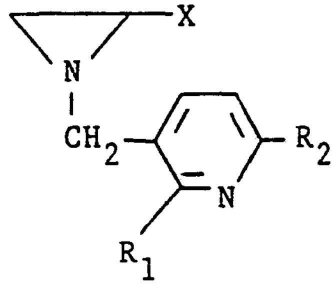

(I)

où

X représente un groupe nitrile,

$R_1$ représente un groupe alcoxy en $C_1$—$C_4$,

$R_2$ représente un groupe hydroxyméthyle, carboxyle, un groupe alcoxy en $C_2$—$C_5$, un groupe formyle ou un groupe carbamoyle leurs sels et leurs N-oxydes.

2. Composés selon la revendication 1, concernant l'acide 5-[(2-cyan-1-aziridinyl)méthyl]-6-méthoxy-pyridine-2-carboxylique et le 1-[(6-hydroxyméthyle-2-méthoxy-3-pyridinyl)méthyl]-2-aziridine-carbonitrile.

3. Procédé pour la préparation des composés de formule générale I

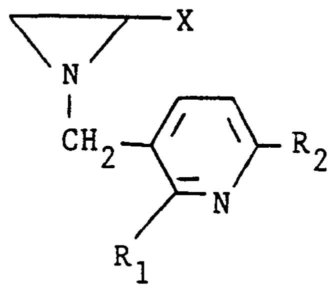

(I)

où

X représente un groupe nitrile,

$R_1$ représente un groupe alcoxy en $C_1$—$C_4$,

$R_2$ représente un groupe hydroxyméthyle, carboxyle, un groupe alcoxy en $C_2$—$C_5$, un groupe formyle ou un groupe carbamoyle, leurs sels et leurs N-oxydes, caractérisé en ce que l'on fait réagir, selon des procédés connus en soi, un composé répondant à la formule générale II

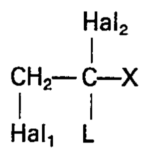

(II),

où X a la signification mentionnée ci-dessus, $Hal_1$ et $Hal_2$ représentent le chlore ou le brome, L représente l'hydrogène, $Hal_1$ et L pouvant représenter également conjointement une valence libre, avec une amine de formule générale III ou son N-oxyde de pyridine

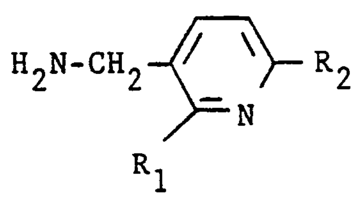

(III)

où $R_1$ et $R_2$ ont les significations mentionnées ci-dessus, et ensuite, on transforme les composés ainsi obtenus, de formule générale I, en d'autres composés de formule générale I, et éventuellement, les composés de formule générale I en leurs sels physiologiquement acceptables.

4. Médicament contenant un des composés selon la revendication 1 ou 2, ainsi que des adjuvants et supports usuels.

5. Utilisation des composés selon la revendication 1 ou 2, pour la préparation de médicaments destinés à lutter contre les maladies liées à un affaiblissement du système immunitaire.

**Claims**

1. Compounds of the general formula I

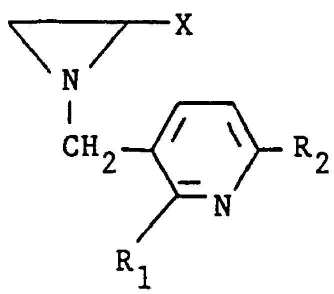

(I)

in which X is a nitrile group, $R_1$ a $C_1$—$C_4$-alkoxy group, $R_2$ the hydroxymethyl, the carboxyl, the $C_2$—$C_5$-alkoxycarbonyl, the formyl or the carbamoyl radical, their salts and N-oxides.

2. Compounds according to claim 1 concerning 5-[(2-cyano-1-aziridinyl)-methyl]-6-methoxypyridine-2-carboxylic acid and 1-[(6-hydroxymethyl-2-methoxy-3-pyridinyl)-methyl]-2-aziridine-carbonitrile.

3. Process for the preparation of compounds of the general formula I

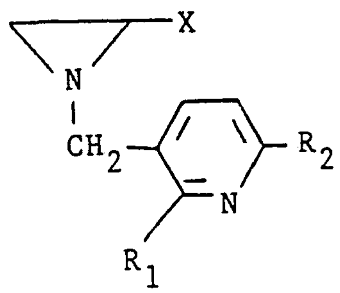

(I)

in which X is a nitrile group, $R_1$ a $C_1$—$C_4$-alkoxy group, $R_2$ the hydroxymethyl, the carboxyl, the $C_2$—$C_5$-alkoxycarbonyl, the formyl or the carbamoyl radical, of their salts and N-oxides thereof, characterised in that, according to per se known methods, one reacts a compound of the general formula II

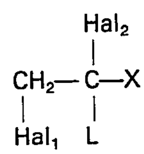

(II)

in which X has the above-given meaning, $Hal_1$ and $Hal_2$ are chlorine or bromine, L signifies hydrogen, whereby $Hal_1$ and L together can also be a valency bond, with an amine of the general formula III or its pyridine-N-oxide

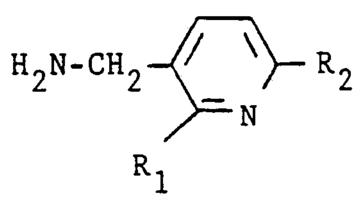

(III)

in which $R_1$ and $R_2$ have the above-given meanings, and subsequently converts the so-obtained compound of the general formula I into other compounds of the general formula I, as well as, if desired, converts the compound of the general formula I into its pharmacologically acceptable salts.

4. Medicaments containing one of the compounds according to claim 1 or 2, besides usual carrier and adjuvant materials.

5. Use of the compounds according to claim 1 or 2 by the preparation of medicaments for the combating of diseases involving a weakening of the immune system.